# EUROPÄISCHE PATENTSCHRIFT

(11) **EP 0 499 147 B1**
(45) Veröffentlichungstag und Bekanntmachung des Hinweises auf die Patenterteilung: **31.01.1996**
(21) Anmeldenummer: 92101989.9
(22) Anmeldetag: 07.02.1992
(51) Int. Cl.: A61M 25/06

(54) **Vorrichtung zur Punktion zum Zweck der Diagnostik und Behandlung von physiologischen und unphysiologischen sowie sonstigen Hohlräumen in den Bereichen der Human- und Tiermedizin**
Puncture device for diagnosis and treatment of physiological, non-physiological or other cavities in human or animal medicine
Dispositif de ponction à fin de diagnostic et traitement physiologique et non physiologique ainsi que d'autres cavités dans le domaine de la médecine humaine ou animale

(30) Priorität: 09.02.1991 DE 4103977
(43) Veröffentlichungstag der Anmeldung: 19.08.1992
(73) Patentinhaber: KENDALL MEDIZINISCHE ERZEUGNISSE GmbH, D-93333 Neustadt (DE)
(72) Erfinder: Meessen, Stefan, Dr. med., D-66424 Homburg/Saar (DE); Piechota, Hansjürgen, Dr. med., 48159 Münster (DE); Brühl, Peter, Prof. Dr. med., 53127 Bonn (DE)
(74) Vertreter: Huber, Arnulf

(56) Entgegenhaltungen:
- EP-A- 0 067 260
- DE-U- 8 524 164
- FR-A- 587 547
- FR-A- 1 225 009
- GB-A- 904 291
- US-A- 3 076 457
- US-A- 3 472 232
- US-A- 4 354 491

## Beschreibung

### I. Bezeichnung der Erfindung

Vorrichtung zur Punktion zum Zweck der Diagnostik und Behandlung von physiologischen und unphysiologischen sowie sonstigen Hohlräumen in den Bereichen der Human- und Tiermedizin.

### II. Patentklasse

Die Erfindung fällt in das Gebiet der medizinischen Heilbehandlung.

### III. Stand der Technik

1. Die medizinisch indizierte Entleerung, Spülung, Auffüllung oder sonstige Behandlung von Hohlräumen - z.B. der Harnblase, des Brustkorbs, des Bauchraumes, des Nierenbeckens, der Gelenke, der Blutgefäße oder von Abszessen und Hämatomen - werden mit handelsüblichen Vorrichtungen zur Punktion (Sets) bestehend aus einem Trokar (Zeichnung 1) oder einer sonstigen Hohlnadel (Kanüle, Braunüle) und einem Schlauch (Katheter, Drain) in der Weise durchgeführt, daß mit Hilfe des Trokars unter Anwendung eines plötzlichen starken manuellen Drucks Haut und Gewebeschichten bis in den Hohlraum durchstochen werden und über den Schlauch ein bleibender Zugang zum Hohlraum geschaffen wird.
2. Die vorliegende Erfindung, die unter anderem in den unter 1. genannten Fällen angewendet werden kann, wird anhand der suprapubischen, perkutanen Harnableitung (SFK) beschrieben. Die SFK-Methode ist eine zwar bekannte, jedoch bis heute nicht allgemein verbreitete Technik. Hierbei wird mit Hilfe eines Trokars (Hohlnadel) unmittelbar über dem Schambein ein Kanal durch Haut, Fettschicht, Faszie, Bauchmuskulatur und Blasenwand bis in das Blaseninnere so gestochen, daß die Blase über einen eingelegten Katheter (Schlauch) entleert, aufgefüllt oder gespült werden kann.
   Die vorbekannten Trokartypen bestehen aus einem Stab (Schaft), der an dem einen Ende eine undefinierte "scharfe Spitze", am anderen einen Griff hat. Vorwiegend sind Trokare gebräuchlich, die
   a) massiv und rund sind und sich im Hohlraum eines Schlauchs befinden (innenliegender Trokar),
   b) hohl und vollrund (Rohr oder Rinne) sind und in der Höhlung den Schlauch aufnehmen (Hohltrokar) oder
   c) massiv und rund sind und von einer zusätzlichen Hülle (Katheterschleuse) umgeben sind, wobei der Schlauch nach der Punktion und nach dem Entfernen des Trokars über dl Hülle in den jeweiligen Hohlraum eingeführt wird (Volltrokar).
3. Die SFK-Methode wird gegenwärtig nur in geringem Maße angewendet,
   a) wenn die Einführung eines zum Stand der Technik gehörenden Harnröhren-Katheters (transurethraler Blasenkatheter) zur Behandlung der Blase nicht möglich ist,
   b) oder wenn die mit einem Harnröhrenkatheter verbundenen Nachteile umgangen werden sollen, wie z.B.
      - Harnröhrenverletzungen (Spätfolge: narbige Einengung),
      - postinstrumentelle Harnröhrenentzündung (Spätfolge: narbige Einengung),
      - Prostata- oder Nebenhodenentzündung, Katheterfieber, Blutvergiftung,
      - kathetervermittelte Harnwegeinfektion,
      - Unmöglichkeit der Restharnprüfung bei liegendem Harnröhrenkatheter,
      - starke subjektive Patientenbelästigung,
      - hoher Aufwand bei der Katheterhygiene.
4. Die SFK-Methode umgeht die Harnröhre und bietet daher gegenüber dem transurethralen Katheter folgende in der Medizin dringend erwünschte Vorteile:
   - Keine postinstrumentelle Harnröhren-, Prostata- und Nebenhodenentzündung,
   - keine narbenbedingte Harnröhreneinengung,
   - Miktion über die Harnröhre bleibt erhalten und bietet die Möglichkeit einer einfachen, exakten Restharnbestimmung,
   - Möglichkeit der Durchführung diagnostischer Maßnahmen (z.B. ante-/retrograde Röntgenuntersuchung der Harnröhre, Blasendruckmessung),
   - Einsatz im Rahmen der Low-pressure-Resektion der Prostata,
   - bessere Patientenverträglichkeit durch geringere Patientenbelästigung und
   - geringer Pflegeaufwand.

Diese Vorteile haben jedoch bisher wegen der entgegenstehenden Nachteile noch nicht zu einer weiten Verbreitung der SFK-Methode in Klinik und Praxis geführt. Die Nachteile bestehen darin, daß die vorbekannten Vorrichtungen zur suprapubischen, perkutanen Blasenpunktion den hohen Anforderungen an ihre Leistungsfähigkeit, an die Anwender- und Anwendungssicherheit sowie an die Patientenfreundlichkeit nicht gerecht werden. Dem Anwender werden ein hohes Maß an Geschicklichkeit und ärztlicher Sorgfalt sowie ein unerwünscht hoher Kraftaufwand abverlangt. Der Patient wird durch den mit Gewalt durchzuführenden Durchstich deutlich beeinträchtigt. Schließlich verursachen herkömmliche Trokartypen durch unphysiologische Schnitteigenschaften unscharfe Einstichwunden ("Sägezahneffekt") und zum Teil Stanzeffekte mit Verschleppung von Hautgewebe in tiefere Gewebeschichten oder in die Blase, was die Asepsis in Frage stellt und - nicht zuletzt durch Urinaustritt - eine Wundinfektion oder eine Harnwegeinfektion entsteht. Diese Nachteile können gegenwärtig nur durch besonders geschickte, auf langen Erfahrungen beruhende Anwendung der SFK-Methode bis zu einem gewissen Grade vermindert werden. Die Nachteile werden dargestellt:
a) in der Beschreibung des US-Patents Nr. 3,860,006 vom 14. Januar 1975 an Hand der dort im einzelnen aufgeführten früheren Patente für suprapubische Kathetersysteme und
b) in der Veröffentlichung von H.J. Piechota, S. Meessen und P. Brühl in "Urologe (B)" 30, S. 195-220 (1990).

Zu a) : In der US-Patentschrift 3,860,006 wird insbesondere das US-Patent Nr. 3,680,562 erwähnt, bei dem ein abgeschrägtes spitzes Durchstich-Element im Inneren einer Katheterröhre angebracht ist, die sich an dem außerhalb des Hohlraums verbleibenden Ende in halbröhrenförmige Teile gabelt, um das Gerät auf der Haut verankern zu können. Als besonderer Nachteil - neben weiteren daselbst aufgeführten Nachteilen - wird hervorgehoben, daß bei diesem Gerät Ablagerungen von Gewebe in der offenen abgeschrägten Spitze des Durchstich-Elements (= Trokar) nicht verhindert werden könnten.

Aber auch das US-Patent 3,860,006 hat diese Nachteile nicht beseitigen können. Es ist nicht in die Produktion gegangen, weil es sich bei der Erprobung im klinischen Alltag nicht bewährt hat. Es hat alle Nachteile der handelsüblichen SFK-Sets in sich vereinigt, die in der vorerwähnten Veröffentlichung in "Urologe (B)" zusammengestellt sind.

Zu b) : In dem erwähnten Artikel in "Urologe (B)", 1990 S. 195 ff. werden insgesamt 12 handelsübliche SFK-Fertigsets auf ihre Vor- und Nachteile bei der suprapubischen Harnblasendrainage untersucht. Im Hinblick auf die hier vorliegende Erfindung ist das Ergebnis der Prüfung von Set Nr. 9 - Vesico Set (Fa. Angiomed) - hervorzuheben, weil es sich dabei um einen rinnenförmigen Trokar mit innenliegendem Katheter handelt, dem jedoch bei der Herstellung des Punktionskanals keine Funktion zukommt. Dieses Punktionsgerät garantiert keine optimalen Schnitteigenschaften, weil die Trokarspitze keine aufgabenspezifischen Schnittwinkel hat. Der Punktionskanal wird lediglich ausgeschnitten und nicht durch gewebeschonende Dilatation geschaffen. Hierdurch werden Stanzeffekte nicht sicher genug vermieden und die Blutungsgefahr nicht ausreichend minimiert.

Es hat immer wieder Bemühungen um Verbesserungen wichtiger Details, insbesondere an Trokar und Katheter gegeben, um eine Weiterverbreitung der vorteilhaften SFK-Methode zu ermöglichen. Jedoch haben bisher alle bekannten Verbesserungversuche nicht zu einer zufriedenstellenden Lösung der medizinischen Anforderungen geführt, die an ein Gerät zur weitverbreiteten, unkomplizierten Anwendung der SFK-Methode zu stellen sind.

Die anhand der SFK-Methode zur Blasenpunktion und Behandlung (Drainage) dargestellten Nachteile bestehen in ähnlicher Weise auch für andere Vorrichtungen zur Punktion zum Zweck der Diagnostik und Behandlung von biologischen Hohlräumen - einschließlich der Blutgefäße (vgl. die Beispiele zu III.1.) - sowie auch für Vorrichtungen zur Funktion von Behältnissen mit medizinischen Flüssigkeiten (z.B. zur Infusion und Irrigation) oder zur Aufnahme von Körperflüssigkeiten auf den Gebieten der Human- und Tiermedizin.

Auch bei diesen Vorrichtungen führt der Ein- und Durchstich mit einer nicht aufgabenspezifisch gestalteten Hohlnadelspitze zu medizinisch unerwünschter Patienbelästigung und erfordert besondere Übung und Geschicklichkeit des Anwenders. Die anhand der SFK-Methode beschriebenen, durch unphysiologische Schnitteigenschaften hervorgerufenen Stanzeffekte mit Verschleppung von Gewebeteilen und der Gefahr nachfolgender Infektionen sind auch bei allen anderen Punktionen verbesserungsbedürftige Nachteile.

Der Einstich in Behältnisse für medizinische Flüssigkeiten hat ebenfalls den Nachteil, daß Bestandteile des Verschlußmaterials in das Innere des Behältnisses verschleppt werden können. Die bisher verwendeten Einstichnadeln mit nicht aufgabenspezifisch gestalteten Spitzen erfordern auch hier unerwünschten Kraftaufwand mit der nachteiligen Möglichkeit technischer Pannen.

In der **GB-A-904,291** ist eine Hohlnadel oder Kanüle für medizinische Zwecke beschrieben, deren Spitze in zwei aneinander anschließenden Bereichen konkav geschliffen ist. Der Übergang zu dem rundum geschlossenen Schaft der Kanüle wird durch eine axial verlaufende Einbuchtung in der Kanülenwand geschaffen, die sich nicht in das Innere der Kanüle fortsetzt und damit den Innendurchmesser nicht reduziert. Die Einbuchtung soll für einen sanften Übergang sorgen und das Ausstanzen von Gewebe oder Material verhindern. Für einen geschlitzten Trokar ist diese bekannte Kanülenspitze jedoch wenig geeignet, da der zur Entnahme eines Katheterschlauchs notwendige Schlitz gerade die dort besonders vorteilhafte Einbuchtung wegfallen ließe.

Aus dem **DE-U-85 24 164** ist ferner ein geschlitzter Trokar bekannt, dessen Spitze allerdings lediglich in einer zur Trokarachse schiefen Ebene abgeschliffen ist.

### IV. Aufgabenstellung

Um der SFK-Methode wegen ihrer bedeutenden medizinischen und pflegetechnischen Vorteile zu weitverbreiteter Anwendung zu verhelfen und bei dieser und anderen Punktionen die vorstehnd genannten Nachteile zu vermeiden, müssen an das aus Trokar und Schlauch bestehende System folgende, bisher noch nicht oder nicht zufriedentellend gelöste Anforderungen gestellt werden:

Die Vorrichtung zur Schaffung des Kanals durch Haut, Fettschicht, Faszie, Muskelgewebe und Hohlraumwand soll
1. optimale Schnitteigenschaften durch eine definierte, scharfkantige, glatte Trokarspitze (dünne Klinge) mit weitestgehend spitzem, kleinem, definiertem Schnittwinkel haben,
2. die Durchstechung der verschiedenen Schichten von der Haut bis in das Innere des Hohlraums (z.B. der Blase) sanft und mit minimaler Kraft des Anwenders ermöglichen,
3. bei der Durchstechung möglichst keine Quetschung oder Stanzeffekte des Gewebes verursachen,
4. durch Schnittform und Glätte der geschaffenen Wundränder eine rasche Zuheilung der Fistel nach Entfernung des Katheters ermöglichen,
5. einen blutungsarmen Punktionskanal schaffen, der eine gewisse Spannung aufweist, um einerseits den Austritt von Flüssigkeit (Urin) aus dem Hohlraum (Harnblase) neben dem durch den Kanal eingeführten Schlauch (Katheter) zu verhindern und andererseits den Eintritt von Mikroorganismen von außen in die Tiefe der Gewebeschichten bis in den Hohlraum zu vermeiden,
6. trotz des erforderlichen extrem spitzen Schnittwinkels der Trokarspitze eine Verletzung oder Durchstechung der gegenüberliegenden Hohlraumwand nach Erreichen des Hohlraums ausschließen,
7. keinen Kalibersprung zwischen Trokar und Katheter haben, um den zu dessen Überwindung notwendigen zusätzlichen Kraftaufwand zu vermeiden.

### V. Lösung der Aufgabenstellung

Zur Lösung der zuvor genannten Aufgabe dient eine Trokarspitze gemäß Patentanspruch 1.

Die Erfindung wird nachfolgend anhand von Zeichnungen näher erläutert; es zeigen:

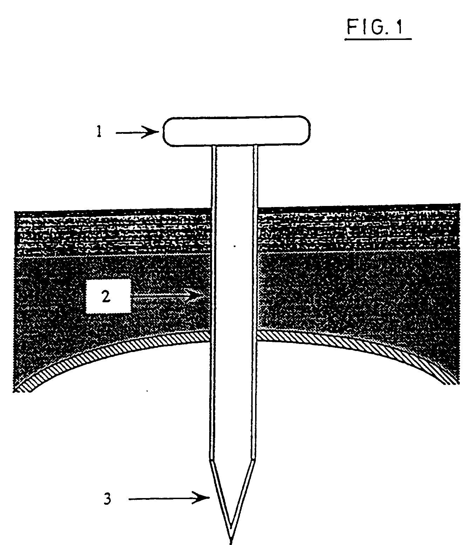

- Fig. 1: das Prizip der Punktion mit einem Trokar;
- Fig. 2a und 2c: Seitenansichten der erfindungsgemäßen Trokarspitze;
- Fig. 2b: eine Draufsicht auf die Trokarspitze aus Fig. 2a;
- Fig. 2d: eine Draufsicht auf eine weniger geeignete Ausführungsform;
- Fig. 3a: das Aufstechen mit der Trokarspitze nach Fig. 2;
- Fig. 3b: das Aufschneiden mit der Trokarspitze nach Fig. 2;
- Fig. 3c: das Aufdehnen mit der Trokarspitze nach Fig. 2; und
- Fig. 3d: das zusätzliche Aufdehnen mit einem in den Trokar eingelegten Katheter.

Verwendet wird ein rinnenförmiger, nicht spaltbarer Hohltrokar (Zeichnung 1), der wie die bekannten Trokarsysteme vorwiegend aus einem Metallstück besteht und in drei Abschnitte, nämlich den - bei einigen Punktionsvorrichtungen entbehrlichen - Griff 1 am proximalen Ende, den Schaft 2 in der Mitte und die Spitze 3 am distalen Ende, gegliedert ist.

### 1. Neue Gestaltung der Trokarspitze

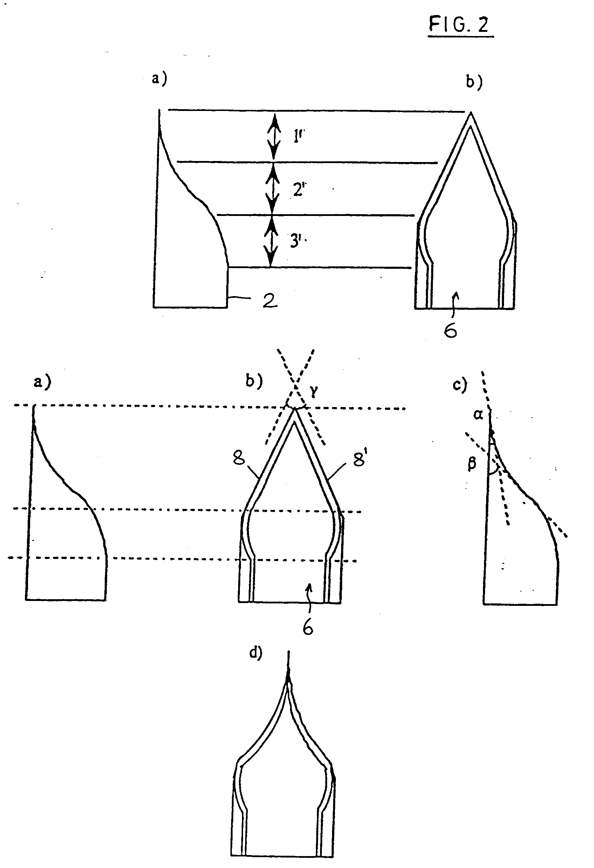

Die Trokarspitze wird schräg und S-förmig (Fig. 2) so zugeschnitten, daß sie drei unterscheidbare Abschnitte erhält, nämlich das (1') Lanzett, das Messer 2' und den Dilatator 3'. Dabei sind die ersten beiden Teile (= 2/3 der Trokarspitze, in der Fig. 2b als "Dorn" bezeichnet) scharf geschliffen, während der letzte Teil, der Dilatator, stumpf bleibt. Vom Lanzett bis zum Dilatator geht die Trokarspitze allmählich in eine Rinnenform über, die in dem Trokarschaft fortgesetzt wird.

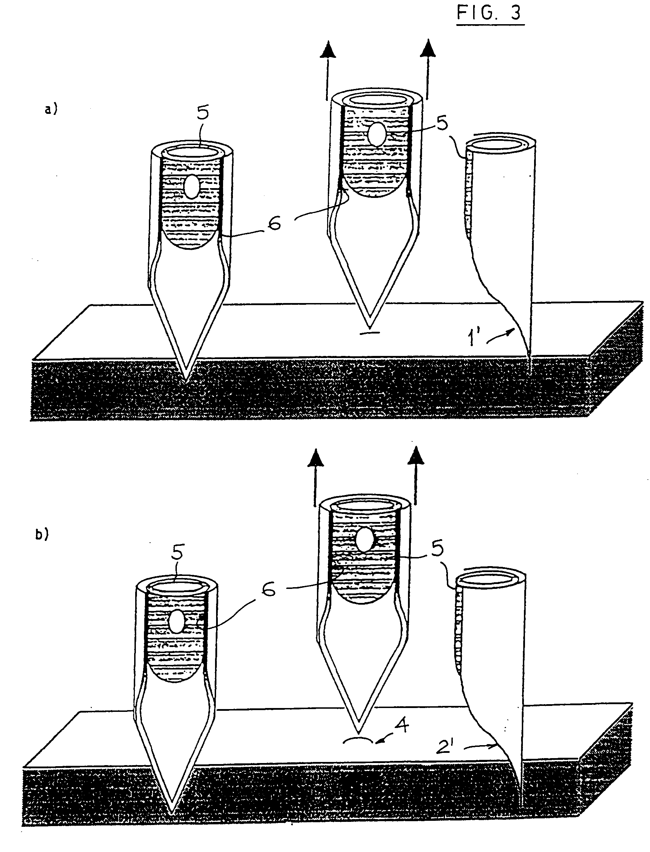

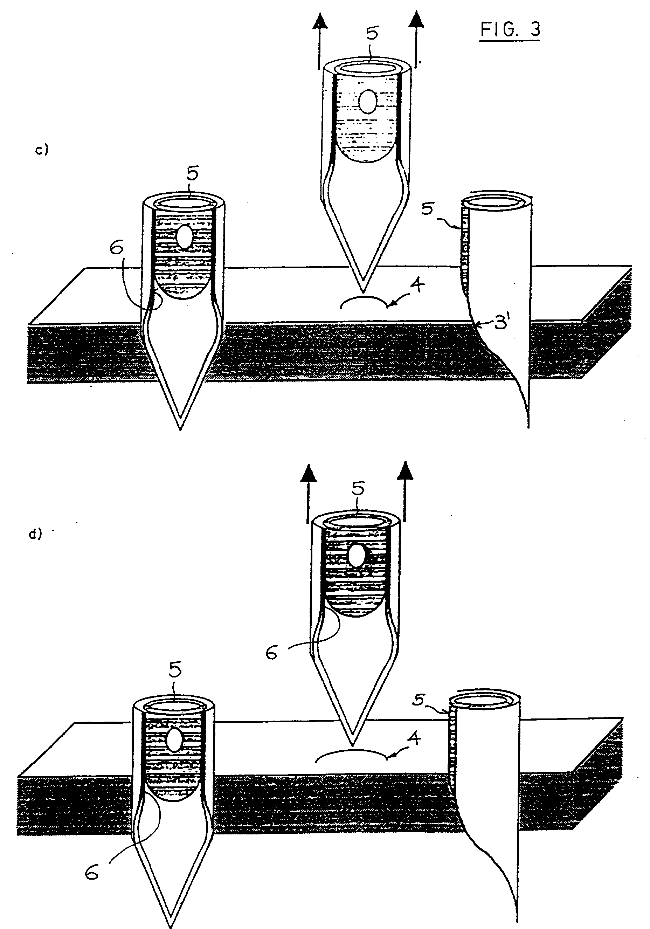

Mit dieser Ausgestaltung der Trokarspitze wird ein Punktionsvorgang bis in den Körperhohlraum in drei Stufen ermöglicht (Fig. 3), nämlich das (a) Aufstechen, das (b) Aufschneiden des durchstochenen Gewebes und die (c) stumpfe, elastische Aufdehnung (Dilatation). Durch diese drei Vorgänge wird eine vollständige Abdichtung des Kanals durch die entstandene innere Hautlippe erreicht. Eine zusätzliche elastische Aufdehnung erfolgt durch den in den rinnenförmigen Trokarschaft eingelegten Schlauch (Fig. 3d). Diese drei Stufen werden wie folgt beschrieben:

### (a) Aufstechen (Fig. 3a)

Das Lanzett 1, mit dem das Gewebe initial aufgestochen wird, ist so spitz und scharf wie möglich angeschliffen, um eine Gewebequetschung zu verhindern und den erforderlichen Kraftaufwand beim Ein- und Durchstich auf ein Minimum zu verringern. Hierzu ist der Schliff so anzulegen, daß das Lanzett in der durch den vorbeschriebenen allmählichen Übergang der Trokarspitze in eine Rinnenform geprägten Seitenansicht eine steile Spitze mit einem Winkel α < 45° (Fig. 2c) bildet.

In Fig. 2d ist in Frontalansicht als zwar naheliegende, aber wegen erheblicher Nachteile abzulehnende Lösung ein Lanzett gezeigt, das im Übergangsteil zum Messer einen konkaven Schliff hat, bei dem die beiden distalen Schnittkanten sehr spitz in einen extrem kleinen Winkel auslaufen. Hierdurch könnte zwar der erwünschte glatte Einstich erreicht werden. Der proximal konkave Zuschnitt des Lanzetts hätte aber zur Folge, daß der Winkel β (Fig. 2c) zwangsläufig vergrößert würde, was die nachfolgende Durchstechung erschweren und einen größeren, dann aber unberechenbaren Kraftaufwand des Anwenders zum Nachteil der ganzen Methode erfordern würde. Denn dieser Kraftaufwand würde die zur Fixierung des Schlauchs notwendige exakte Ermittlung der Breite des Schlitzes Z im Trokarschaft erheblich erschweren. Damit würde durch die Mitwirkung des Schlauchs beim Dilatationsvorgang eine Spannung im Bereich der Haut und des Punktionskanals herzustellen, die Blutungen und einen Austritt von Flüssigkeit aus dem punktierten Hohlraum vermeidet, weitgehend ausgeschlossen werden. Beim konkaven Zuschnitt des Lanzetts würde also ein Teil der erstrebten Vorteile durch erhebliche Nachteile aufgewogen werden; er ist deshalb nicht zu empfehlen.

Erfindungsgemäß wird daher von konkaven Schnittkanten abgesehen und - wie in Fig. 2b dargestellt wird - ein gerader Schliff der Lanzettspitze so gewählt, daß einerseits der Winkel des Lanzetts so klein wie möglich wird, andererseits aber die angeschliffenen Kanten 8,8' der Lanzettspitze noch als "gerade" und nicht als konkav angesehen werden können. Der Winkel muß, um eine optimale Minderung der bei der Punktion aufzuwenden Kraft zu erreichen, kleiner als 45° sein und möglichst nahe an 30° herankommen, darf jedoch nicht darunter liegen.

### (a) Aufschneiden (Fig. 3b)

Das Aufschneiden des angestochenen Gewebes in der zweiten Stufe des Punktionsvorgangs erfolgt mit dem zweiten Drittel der Trokarspitze 2' (Fig. 2a/b), das weniger steil als das Lanzett und mit je einem angeschliffenen Messer auf jeder Seite ausgestaltet ist. Diese Messer weisen in ihrer Seitenansicht, die durch die allmähliche Ausformung zur Rinnenform bedingt ist, einen Applikationswinkel von β = 45° (Fig. 2c) auf. Der Winkel β ist in dieser Größe zu wählen, um zu bewirken, daß die Haut nicht aufgerissen, sondern ohne Hauteinrisse ("Sägezahneffekt") glatt durchschnitten wird, was für die Infektionsprophylaxe und die Verminderung des zur Punktion erforderlichen Kraftaufwands von ausschlaggebender Bedeutung ist.

Die bis in die Trokarspitze fortgeführte Rinnenform des Trokarschafts bewirkt, daß der mit dem zweiten Drittel der Trokarspitze ausgeführte Schnitt nicht - wie bei den bekannten rinnenförmigen Trokaren - einen fast vollen Kreis, sondern nur den in Fig. 3b dargestellten Halbkreis 4 ergibt. Hierdurch werden ein völliges Ausstanzen der Haut und des darunter gelegenen Gewebes und damit ein Verschleppen von epidermalem Gewebe und Bakterien in tiefere Gewebeschichten oder in den Hohlraum mit Sicherheit vermieden und auch die schnittbedingte Blutung minimiert.

### (c) Stumpfes Aufdehnen (Fig. 3c)

Die in Stufe 2 halbkreisförmig geschnittene Lippe wird durch das letzte Drittel der Trokarspitze, den Dilatator 3' (auch als "Bougierdrittel" bezeichnet), aufgedehnt. Dieser hat stumpfe Kanten und ist in der vom Trokarschaft her fortgeführten Rinnenform in der Seitenansicht S-förmig. Durch diese Ausgestaltung vermeidet er ein weiteres Aufschneiden oder Ausstanzen der gebildeten Lippe und drängt stumpf und elastisch sowie zugleich blutungsstillend die Wundränder des Punktionskanals halbmondförmig auseinander. Die Wundlippen werden elastisch so weit gedehnt, daß sie eng anliegend den Trokarschaft hindurchlassen und nach Entfernung des Trokars den Schlauch dicht umschließen.

Der Dilatator 3' geht rund und glatt in den Trokarschaft 2 über, um ein schlagartiges Abfallen des Gewebewiderstands zu vermeiden. Hierdurch wird die Gefahr minimiert, daß durch zu großen Kraftaufwand zu weit durchgestochen und die gegenüberliegende Blasenwand verletzt wird.

### 2. Mitwirkung des Schlauchs bei der Dilatation

Bei allen bekannten Hohltrokaren und auch bei dem in "Urologe (B)" 1990, S. 199 ff. beschriebenen Rinnentrokar (Set 9) liegt der in den ausgestanzten Punktionskanal einzuführende Schlauch zwar im Inneren des Trokarschafts, beteiligt sich aber nicht an dem zu 1.c beschriebenen stumpfen Aufdehnen. Er wird vielmer erst nach Beendigung des Durchstichs durch die Trokarspitze hindurch in den Hohlraum hineingeschoben. Das hat den Nachteil, daß der Durchmesser des Punktionskanals stets geringfügig größer ist als das Kaliber des Schlauchs. Dies führt nach dem Entfernen des Trokars zu Abdichtungsproblemen und damit zur Gefahr von Blutung, Urinaustritt und Infektion.

Zur Vermeidung dieser Nachteile wird der Schlauch 5 vor dem Punktionsvorgang in den Trokar so eingelegt, daß er mit dem später in den Hohlraum einzuführenden Ende bis in den Dilatator der Trokarspitze hineinreicht und dort durch dessen sigmoidförmigen übergang zur Rinnenform des Trokarschafts festgehalten wird. Der so eingelegte Schlauch 5 ragt - wie aus den Seitenansichten in Fig. 3 ersichtlich ist - teilweise aus dem Dilatator und dem Schlitz 6 des Trokarschafts heraus.

Hierdurch wird erreicht, daß sich der Schlauch 5 an der Aufdehnung der ausgeschnittenen inneren Hautlippe und des nachfolgend geschaffenen Punktionskanals beteiligt (Fig. 3d, Seitenansicht). Diese Beteiligung hat zur Folge, daß sich die aufgeschnittene Hautlippe und die Wand des Punktionskanals nach dem Entfernen des Trokars eng und damit blutungsstillend und wasserdicht an den Schlauch anlegen und den Austritt der im punktierten Hohlraum (z.B. Blase) befindlichen Flüssigkeit (z.B. Urin) in das umgebende Gewebe und weiter nach außen mit möglicher Entstehung z.B. einer Urinphlegmone verhindern. Umgekehrt wird auch das Eindringen von Mikroorganismen von außen her erschwert. Wesentliche Voraussetzung für diese Mitwirkung des Schlauchs an der neuen Art der Aufdehnung des Punktionskanals mit dichtem Anschluß der Schlauchwand an die Wand des Punktionskanals ist der feste Sitz des Schlauchs im Trokarschaft.

Nachdem der Trokar durch alle Gewebsetagen (z.B. Haut-Unterhaut-Fettgewebe-Faszie-Muskulatur-Harnblasenwand) hindurch in den zu punktierenden Hohlraum (z.B. die Harnblase) eingeführt ist, wird der darin befindliche Schlauch durch den liegenden (ruhenden) Trokar weiter in den Hohraum vorgeschoben, bis er in diesen in ausreichender Länge hineinragt. Sodann wird er durch den für diesen Zweck vorgesehenen Schlitz des Trokarschafts um 90° abgekippt und damit vom Trokar gelöst, so daß dieser unter Zurücklassung des Schlauchs herausgezogen werden kann.

Die neue Vorrichtung zur Schaffung eines Punktionskanals in den vorbeschriebenen drei Phasen und die wesentliche Mitwirkung des Schlauchs beim Dilatationsvorgang bewirken, daß sich die beiden dabei gebildeten Wundränder bei der Entfernung des Katheters wieder zusammenziehen können, weil die sogenannte "elastische Phase" der Haut durch die erfindungsgemäß schonende Abfolge von stufenweisem Durchstechen, Aufschneiden und Aufdehnen während des Punktionsvorgangs weitgehend erhalten geblieben ist. Hierdurch wird die spätere Wundheilung gefördert.

## Patentansprüche

1. Trokarspitze für einen über seine ganze Länge einen Schlitz (6) aufweisenden Trokar, dessen Schaft (2) zur Bildung der Spitze (3) im Winkel zur Trokarachse abgeschrägt ist,
**dadurch gekennzeichnet**,
- daß die Spitze (3) aus drei gleich langen Teilen besteht, nämlich dem Lanzett (1'), dem Messer (2')und dem Dilatator (3'), die ohne Sprung ineinander und in den Schaft (2) übergehen, wobei die Spitze (3) vom Lanzett (1') zum Dilatator (3') rinnenförmig gebogen ist;
- daß das Lanzett (1') in der Seitenansicht einen Neigungswinkel α von weniger als 45° und in der Draufsicht einen Spitzenwinkel γ von 30° - 45° hat, der von geraden Schenkeln (8, 8') gebildet ist;
- daß das Messer (2') einen Neigungswinkel β von mehr als 45° und scharf geschliffene Schneiden bildet, die sich zunehmend zu der Rinnenform nähern; und
- daß der Dilatator (3') abgerundete Oberkanten aufweist, die S-förmig in die Ränder des Trokarschlitzes (6) übergehen.

## Claims

1. A trocar tip for a trocar which exhibits over its whole length a split (6) and the shank (2) of which is bevelled at an angle to the axis of the trocar for the formation of the tip 93), characterized in that
- the tip (3) consists of three parts of equal length, namely, the lancet (1'), the knife (2') and the dilator (3'), which continue into one another and into the shank (2) without a break, the tip (3) being curved from the lancet (1') to the dilator (3') in the shape of a trough;
- the lancet (1') has in side elevation an angle of inclination α of less than 45° and in plan an angle γ at the tip of 30° - 45°, which is formed by straight sides (8, 8');
- the knife (2') forms an angle of inclination β of more than 45° and sharply ground cutting edges which approach increasingly to the trough shape; and
- the dilator (3') exhibits rounded top edges which continue into the edges of the split (6) in the trocar, in the shape of a S.

## Revendications

1. Pointe de trocart pour un trocart comportant une fente (6) sur toute sa longueur, dont la tige (2) est biseautée en formant un angle sur l'axe du trocart pour la formation de la pointe (3) caractérisée en ce que :
- la pointe (3) est constituée de trois parties de longueurs égales, a savoir la lancette (1'), le couteau (2') et le dilatateur (3'), qui se succèdent, l'un l'autre et se raccordent a la tige sans discontinuité, la pointe (3) étant incurvée en forme de gouttière depuis la lancette (1') jusqu'au dilatateur (3'),
- la lancette (1') présente en vue latérale un angle d'inclinaison α de moins de 45 degrés et de dessus en plan un angle au sommet γ compris entre 30 et 45 degrés, qui est délimité par des côtés droits (8, 8').
- le couteau (2') a un angle d'inclinaison (β) supérieur a 45 degrés et forme des lames biseautées de manière tranchante qui se rapprochent progressivement de la forme d'une gouttière, et
- le dilatateur (3') comporte des bords supérieurs arrondis qui se raccordent en forme de S aux extrémités de la fente (6) du trocart.
